# FASCICULE DE BREVET EUROPEEN

(11) **EP 2 408 512 B1**
(45) Date de publication et mention de la délivrance du brevet: **22.10.2014**
(21) Numéro de dépôt: 10709501.0
(22) Date de dépôt: 17.03.2010
(51) Int. Cl.: A61M 39/10, F16L 37/56

(54) **ADAPTATEUR POUR RACCORDER UN CONNECTEUR DE RÉCIPIENT À UNE PRISE DE RACCORD D'UNE MACHINE DE DIALYSE**
ADAPTER ZUR VERBINDUNG EINES BEHÄLTERKONNEKTORS MIT EINER ANSCHLUSSBUCHSE EINES DIALYSEGERÄTS
ADAPTER FOR CONNECTING A CONTAINER CONNECTOR TO A CONNECTION SOCKET OF A DIALYSIS MACHINE

(30) Priorité: 20.03.2009 FR 0951791
(43) Date de publication de la demande: 25.01.2012
(73) Titulaire: Fresenius Medical Care Deutschland GmbH, 61352 Bad Homburg (DE)
(72) Inventeur: GASTAUER, Paul, Hongkong (CN); LAFFAY, Philippe, F-69110 Sainte Foy Les Lyon (FR); FAYE, Bruno, F-69490 Saint Forgeux (FR)
(74) Mandataire: Vièl, Frédérique
(86) Numéro de dépôt international: PCT/EP2010/053435
(87) Numéro de publication internationale: WO 2010/106091

(56) Documents cités:
- EP-A- 1 344 550
- DE-A1- 4 303 372
- FR-A- 2 766 797
- GB-A- 1 360 732
- US-A1- 2004 194 918

## Description

L'invention concerne un adaptateur pour raccorder un connecteur de récipient à une prise de raccord d'une machine de dialyse, le connecteur étant muni de deux lignes de fluide reliant l'intérieur et l'extérieur du récipient dont les extrémités ouvertes vers l'extérieur sont concentriques, la prise de raccord de la machine de dialyse étant munie de deux lignes de fluide dont les extrémités ouvertes vers l'extérieur sont non concentriques et éloignées l'une de l'autre.

De nombreuses machines de dialyse sont équipées de moyens pour fabriquer elles-mêmes les solutions de dialyse à partir d'eau et d'un concentré. Ce concentré peut se présenter sous la forme d'une solution concentrée ou d'un produit solide pulvérulent ou granuleux. Il s'agit en général de sels. Pour la réalisation de la solution, la machine doit introduire de l'eau dans la poche et la solution ainsi réalisée doit être aspirée par la machine, puis diluée.

Les cartouches ou les poches sont donc munies d'un connecteur dont la forme est complémentaire à celle de la fiche de raccord de la machine de dialyse. Malheureusement, chaque type de machine de dialyse a sa propre fiche de raccord, même dans une gamme de machines d'une même société. Il s'ensuit que les cartouches ou les poches, contenant un même produit, doivent être proposées dans différentes formes, une même poche n'est pas forcément compatible avec deux machines différentes.

Le document WO 2007/082548 propose ainsi une cartouche avec deux jeux de connecteurs, l'un pour une machine de la marque Gambro et l'autre pour une machine de la marque Fresenius. Par conséquent, une même cartouche peut être utilisée sur deux machines différentes. Cependant, chaque cartouche n'est utilisée qu'une fois sur l'une ou l'autre des machines, de sorte que seul l'un des jeux de connecteurs sera effectivement utilisé. Cela implique donc des frais supplémentaires importants.

Parmi les machines de la société Fresenius, on connaît des machines travaillant avec des connecteurs avec des lignes de fluide concentriques et des machines avec des lignes de fluide séparées. Dans le cas des connecteurs concentriques, dont le principe de fonctionnement est présenté dans les documents DE 43 03 372 A1 et FR 2 766 797 A1, les lignes de fluide, servant à amener l'eau et à aspirer ensuite la solution, ont leurs extrémités dirigées vers l'extérieur du flacon concentrique. Cela se traduit par une première ligne de fluide débouchant dans une première chambre circulaire délimitée par une première nervure et une deuxième ligne de fluide débouchant dans une chambre annulaire située autour de la première chambre. Cette deuxième chambre est définie par la première nervure et par une seconde nervure située autour de façon concentrique. Ces connecteurs sont en général placés sur l'une des faces verticales de la cartouche ou de la poche.

La fiche de raccord de la machine de dialyse a une forme correspondante pour réaliser après montage la continuité des lignes de fluide du récipient et de la machine.

Dans le cas des lignes de fluide séparées, dont le principe est présenté dans le document EP 1 344 550 A1, le connecteur est muni de deux lignes de fluide séparées dont les extrémités extérieures se finissent par deux embouts qui viennent s'introduire dans deux orifices correspondants de la machine de dialyse. Les embouts sont placés à distance l'un de l'autre et assurent la jonction entre les lignes de fluide du connecteur et celles de la machine. Les deux sorties ne sont plus concentriques mais parallèles.

L'objectif de l'invention est de développer un moyen pour permettre l'utilisation de cartouches ou de poches munies d'un connecteur concentrique sur une machine à dialyser ayant une fiche de raccord avec des lignes de fluide séparées.

Cet objectif est atteint conformément à l'invention du fait que l'adaptateur est muni d'une première et d'une seconde nervure d'adaptateur concentriques, la première formant une chambre circulaire ouverte vers l'extérieur dans le fond de laquelle est réalisé un premier orifice tandis que la deuxième, placée autour de la première, forme une chambre annulaire ouverte vers l'extérieur dans le fond de la quelle est réalisé un deuxième orifice, l'adaptateur étant en outre muni d'un premier canal de liaison dans lequel débouche le premier orifice provenant de la première chambre circulaire et d'un deuxième canal de liaison isolé du premier dans lequel débouche le deuxième orifice provenant de la deuxième chambre annulaire, chaque canal comprenant un embout, lesdits embouts étant dimensionnés et disposés de telle sorte qu'ils peuvent être introduits dans les éléments de réception de la prise de raccord de la machine de dialyse. Grâce à cet adaptateur, il est possible de transformer une sortie de lignes de fluide concentriques en une sortie de lignes de fluide parallèles.

Afin de faciliter la mise en place de l'adaptateur sur la prise de raccord parallèle, les axes des embouts sont parallèles et les extrémités des embouts sont disposées dans un même plan perpendiculaire aux axes des embouts. Ainsi, il sera très simple d'enficher les deux embouts du haut vers le bas dans les orifices récepteurs correspondants de la fiche de raccord de la machine.

Lorsque l'adaptateur est destiné à une poche ou un récipient ayant un connecteur placé sur une des ses faces verticales, les axes des embouts sont perpendiculaires à l'axe des nervures concentriques. Ainsi, en position montée, la poche conserve sa position verticale.

Conformément à l'invention, la première nervure d'adaptateur et la deuxième nervure d'adaptateur sont dimensionnées de telle sorte que lorsque l'adaptateur est monté sur un connecteur concentrique, elles viennent s'emboîter sur ou dans des première et deuxième nervures correspondantes du connecteur de sorte que les deux chambres concentriques sont isolées l'une de l'autre et vis-à-vis de l'extérieur.

Afin que l'adaptateur ne se décroche pas du connecteur, des moyens de crochetage sont prévus pour en position d'utilisation retenir l'adaptateur sur le connecteur. Ces moyens de crochetage sont de préférence constitués par au moins un arceau monté sur des crochets.

Dans un mode de réalisation particulier de l'invention, les canaux ne sont pas alignés et forment un angle obtus entre eux.

Afin de permettre de soulever le récipient lorsqu'il est muni de l'adaptateur, il est préférable de munir ce dernier d'une poignée.

Il est préférable de munir l'adaptateur de moyens de retenue, notamment une plaque d'appui, destinés à coopérer avec des moyens complémentaires sur la machine de dialyse, notamment un couvercle, pour empêcher l'adaptateur de pivoter sous l'effet du poids lorsqu'il est monté dans la machine.

Dans un mode de réalisation privilégié de l'invention, l'adaptateur est muni d'une plaque de détection destinée à coopérer avec des moyens de détection complémentaires sur la machine de dialyse

L'invention concerne également un set constitué d'un adaptateur selon l'une des revendications précédentes et d'un récipient muni d'un connecteur pour raccorder le récipient à une prise de raccord d'une machine de dialyse, le connecteur étant muni de deux lignes de fluide reliant l'intérieur et l'extérieur du récipient dont les extrémités ouvertes vers l'extérieur sont concentriques. Le récipient peut être par exemple une poche ou une cartouche.

L'invention est décrite plus en détail à l'aide de figures suivantes qui montrent :

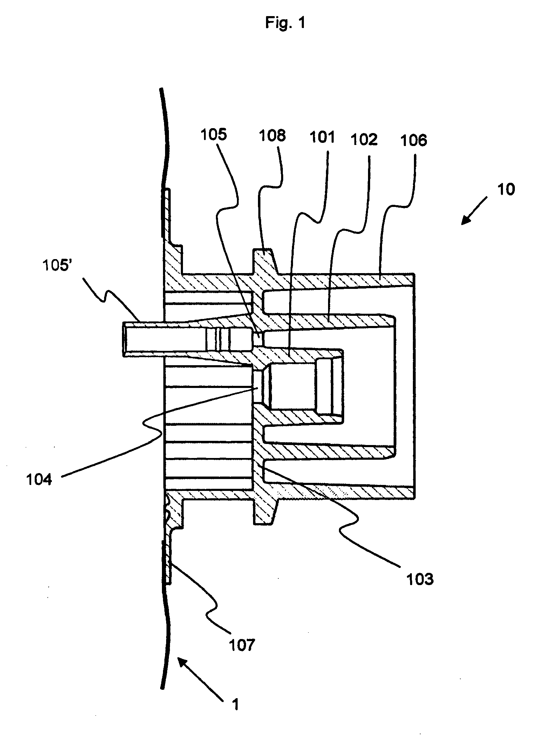

- Figure 1 :: une vue en coupe d'un connecteur circulaire ;

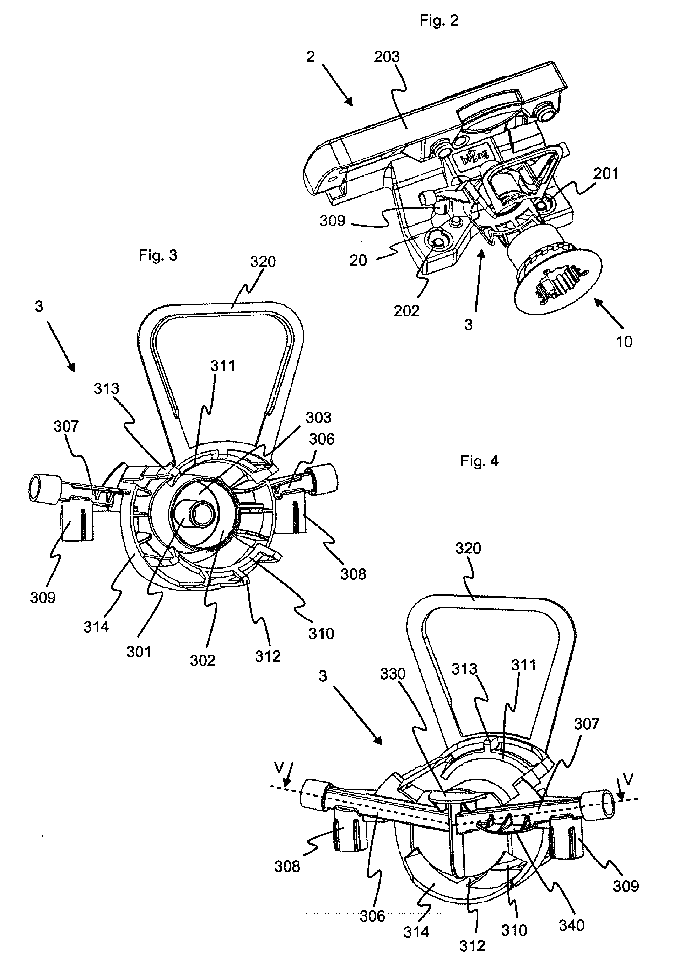

- Figure 2 :: une vue en perspective d'une prise de connexion d'une machine de dialyse à lignes de fluide parallèles ;
- Figure 3 :: une vue en perspective de face d'un adaptateur selon l'invention ;
- Figure 4 :: une vue en perspective de dos d'un adaptateur selon la figure 3 ;

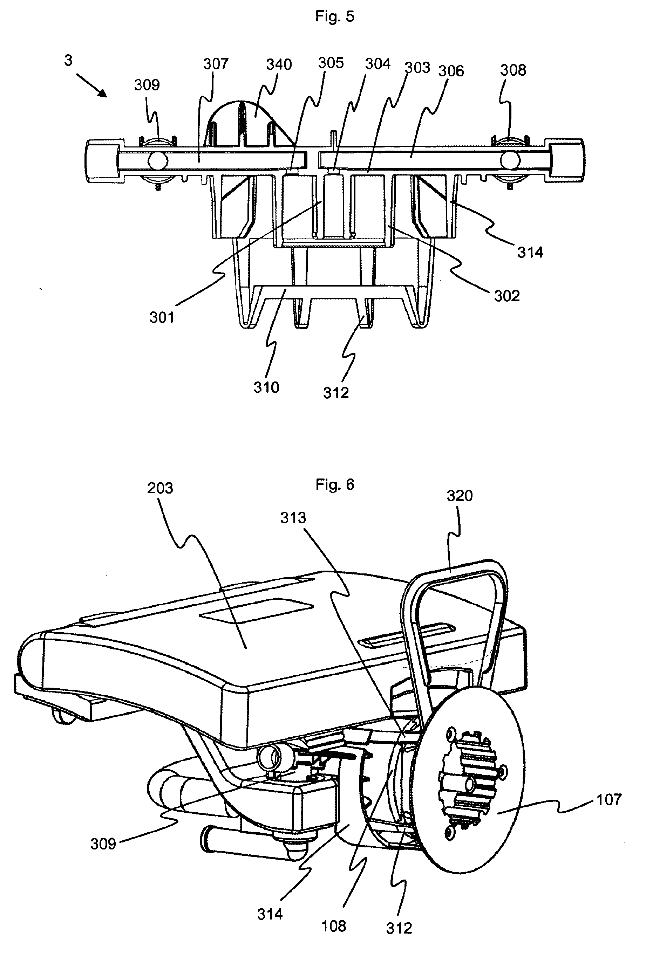

- Figure 5 :: une vue en coupe au travers des canaux de liaison de l'adaptateur de la figure 3 selon la ligne V-V de la figure 4 ;
- Figure 6 :: une vue en perspective d'un adaptateur selon la figure 3, monté sur un connecteur circulaire de la figure 1 et placé dans une machine de dialyse de la figure 2.

L'adaptateur de l'invention a pour objectif de permettre l'usage d'un récipient muni d'un connecteur concentrique sur un appareil de dialyse destiné à recevoir des récipients munis de connecteurs avec des entrées de lignes de fluide séparées et distantes.

Un connecteur concentrique (10) est constitué essentiellement de deux nervures de connecteur (101, 102) concentriques placées perpendiculairement sur une base circulaire (103). Des orifices (104, 105) sont pratiqués dans la base circulaire (103). Ces orifices peuvent se prolonger à l'intérieur du récipient par des tuyaux plongeant dans le récipient (1), tels que la tubulure (105'). Le premier orifice (104) est placé à l'intérieur de la première nervure (101), nervure centrale, et le deuxième dans l'espace compris entre la première (101) et la seconde (102) nervure. Le connecteur circulaire est donc muni de deux lignes de fluides définies chacune par au moins un orifice (104, 105) et un canal formé pour le premier par l'espace intérieur de la première nervure (101) et pour le second par le canal annulaire défini par l'espace situé entre la première (101) et la deuxième (102) nervure. Du côté extérieur, les extrémités à raccorder des deux lignes de fluide sont donc concentriques.

En général, le connecteur concentrique comprend en outre une troisième nervure (106), concentrique aux deux autres (101, 102) et placée à l'extérieur de celles-ci, laquelle se prolonge au-delà de la base (103) et se termine par une collerette (107) sur laquelle est soudé le récipient (1). Pour permettre une fixation sûre du connecteur sur l'appareil de dialyse, il est également prévu une seconde collerette (108) sur laquelle s'accrocheront des crochets fixés à la machine de dialyse. Cette seconde collerette (108) est placée sur le côté extérieur du connecteur par rapport à la collerette (107) pour la soudure du récipient.

La fiche de raccord (20) d'une machine de dialyse avec deux lignes de fluide séparées parallèles comprend deux orifices verticaux (201, 202) dans lesquels débouchent des lignes de fluide menant à l'intérieur de la machine et non représentées. Ces orifices verticaux (201, 202) sont parallèles et situés dans un même plan horizontal à une distance prédéfinie sur les extrémités d'un U. Pour la machine à dialyse 5008 de Fresenius, cette distance est de l'ordre de 70 mm. Ces orifices servent d'une part de raccord pour les lignes de fluides du connecteur du récipient et celles de la machine et d'autre part de support pour le connecteur du récipient. Un couvercle (203) se rabat sur le connecteur pour le maintenir en place.

L'adaptateur de l'invention est donc conçu pour séparer les lignes de fluides concentriques du connecteur et obtenir deux lignes de fluides séparées se terminant chacune par un embout dont les dimensions et les emplacements soient compatibles avec la prise de connexion de l'appareil de dialyse.

L'adaptateur (3) de l'invention est constitué d'une plaque de base (303) de laquelle partent perpendiculairement deux nervures d'adaptateurs concentriques : la nervure centrale (301), appelée première nervure, et la nervure externe (302), dite deuxième nervure. Il y a donc une première chambre, circulaire, délimitée d'une part par la première nervure (301) et d'autre part par la plaque de base (303), et une deuxième chambre, annulaire, délimitée d'une part par la première (301) et la deuxième (302) nervure et d'autre part par la plaque de base (303). Ces deux chambres sont ouvertes vers l'extérieur, c'est-à-dire du côté par lequel est introduit le connecteur (10).

La plaque de base (303) est munie de deux orifices (304, 305) et de deux canaux (306, 307) qui partent sensiblement du centre de la plaque de base et vont en s'écartant. Les canaux sont de préférence placés sur la face de la plaque de base (303) opposée aux nervures (301, 302).

Le premier orifice (304) relie la première chambre, c'est-à-dire l'espace compris à l'intérieur de la première nervure d'adaptateur (301), et le premier canal (306) tandis que le second (305) relie le second canal (307) et la deuxième chambre, donc l'espace situé entre la première (301) et la seconde (302) nervure d'adaptateur. Les deux canaux ne communiquent pas. À distance de la plaque de base (303), chaque canal (306, 307) est muni d'un embout (308, 309) dont les dimensions sont compatibles avec celles d'une machine de dialyse à lignes de fluide séparées. Dans l'exemple présenté, les embouts (308, 309) sont perpendiculaires aux nervures (301, 302). Cela permet donc de relier à la machine un récipient dont le connecteur (1) se situe sur une de ses faces verticales.

Les dimensions et les positions de ces nervures d'adaptateur (301, 302) par rapport à la plaque de base (303) sont choisies de telle sorte qu'elles correspondent aux nervures de connecteur (101, 102) du connecteur (10) du récipient. Par exemple, la première nervure d'adaptateur (301) est dimensionnée pour pénétrer dans l'espace défini par la première nervure de connecteur (101) en formant une liaison étanche. La deuxième nervure d'adaptateur (302) vient entourer la deuxième nervure de connecteur (102) en formant une liaison étanche. En d'autres termes, la première chambre et la deuxième chambre, initialement ouverte vers l'extérieur, sont maintenant fermées par le connecteur et les lignes de fluide du connecteur (104, 101 ; 105, 102) se prolongent par les lignes de fluide de l'adaptateur (301, 304, 306, 308 ; 302, 305, 307, 309).

Afin de bloquer l'adaptateur (3) sur le connecteur (1), il est prévu un dispositif de crochetage (310, 311) constitué par exemple de deux arceaux (310, 311) reliés par plusieurs crochets (312, 313) à la plaque de base (303) via une troisième nervure concentrique (314). Lorsque l'adaptateur (3) est monté sur un connecteur concentrique (1), ces moyens de crochetage (310, 311) viennent en prise derrière la collerette (108) du connecteur. Grâce à l'inclinaison des extrémités repliées des crochets (312, 313), il est facile d'enfiler un adaptateur sur le connecteur, les crochets s'écartant lorsque leurs extrémités repliées viennent glisser contre le bord périphérique de la collerette (108). Mais une fois les arceaux (310, 311) placés derrière cette collerette (108), il n'est plus possible de ressortir l'adaptateur par inadvertance sans casser les crochets (312, 313) ou les arceaux (310, 311).

Afin de bloquer l'adaptateur dans la machine de dialyse, celle-ci est munie d'un couvercle (203) qui vient appuyer sur une plaque d'appui (330) située sur la plaque de base (303), au-dessus des deux canaux (306, 307), perpendiculairement à la plaque de base (303) et parallèlement à l'axe des nervures (301, 302). Ainsi, le flacon, en appui par ses embouts (308, 309) enfichés dans les récepteurs correspondants (201, 202) de la machine est empêché de basculer en arrière par ce blocage de la plaque d'appui (330) par le couvercle (203).

Pour permettre de soulever le récipient muni d'un adaptateur selon l'invention, il est prévu de munir ce dernier d'une poignée (320).

Pour permettre la détection de la présence du récipient muni de l'adaptateur, il est prévu de muni ce dernier (3) d'une plaque de détection (340). Lors de la mise en place du récipient muni de l'adaptateur, la machine pourra détecter sa présence grâce à un détecteur placé convenablement.

Il est préférable que les canaux (306, 307) ne soient pas alignés, mais forment un angle obtus entre eux. Dans l'exemple présenté aux figures, cet angle est de l'ordre de 162°. Autrement dit, les canaux remontent en s'écartant du centre de l'adaptateur en formant un angle d'environ 9° par rapport à l'horizontale.

Pour la fabrication de l'adaptateur, tous les matériaux adaptés au moulage par injection et autorisés par la pharmacopée peuvent être utilisés. On citera à titre d'exemple le polyéthylène, le polypropylène, le polysulfone, le polyacétale et le polyoxyméthylène (POM).

Grâce à l'adaptateur conforme à l'invention, il est possible d'utiliser des cartouches ou des poches munies d'un connecteur concentrique sur une machine à dialyser ayant une fiche de raccord avec des lignes de fluide séparées. Cela permet de diminuer le stock de récipients en se limitant à des récipients munis de connecteurs concentriques.

L'adaptateur de l'invention peut être fourni séparément ou dans un set avec un récipient contenant le produit à dissoudre, tel que par exemple une poche ou une cartouche. Dans ce cas, il est préférable de ne pas le monter d'office sur le connecteur du récipient pour laisser le choix à l'utilisateur final de le monter ou non en fonction de la machine de dialyse utilisée.

### Liste de références :

- 1: Récipient
- 10: Connecteur
- 101: Première nervure de connecteur
- 102: Deuxième nervure de connecteur
- 103: Base
- 104: Premier orifice
- 105: Deuxième orifice
- 105': Tubulure prolongeant le deuxième orifice
- 106: Troisième nervure de connecteur
- 107: Collerette de fixation du récipient
- 108: Collerette de fixation de l'adaptateur

- 2: Machine à dialyser
- 201: Premier orifice de réception de la fiche de raccord
- 202: Deuxième orifice de réception de la fiche de raccord
- 203: Couvercle

- 3: Adaptateur
- 301: Première nervure d'adaptateur
- 302: Deuxième nervure d'adaptateur
- 303: Plaque de base
- 304: Premier orifice
- 305: Deuxième orifice
- 306: Premier canal
- 307: Deuxième canal
- 308: Premier embout
- 309: Deuxième embout
- 310: Premier arceau de crochetage
- 311: Deuxième arceau de crochetage
- 312: Premier jeu de crochets
- 313: Deuxième jeu de crochets
- 314: Troisième nervure d'adaptateur
- 320: Poignée
- 330: Plaque d'appui
- 340: Plaque de détection

## Revendications

1. Adaptateur (3) pour raccorder un connecteur (10) de récipient à une prise de raccord (2) d'une machine de dialyse, le connecteur (10) étant muni de deux lignes de fluide reliant l'intérieur et l'extérieur du récipient dont les extrémités ouvertes vers l'extérieur sont concentriques, la prise de raccord (2) de la machine de dialyse étant munie de deux lignes de fluide dont les extrémités ouvertes vers l'extérieur forment des orifices (201, 202) qui sont non concentriques et éloignés l'un de l'autre d'une distance prédéfinie, l'adaptateur (3) étant muni d'une première (301) et d'une seconde nervure d'adaptateur (302) concentriques, la première formant une chambre circulaire ouverte vers l'extérieur dans le fond de laquelle est réalisé un premier orifice (304) tandis que la deuxième, placée autour de la première, forme une chambre annulaire ouverte vers l'extérieur dans le fond de la quelle est réalisé un deuxième orifice (305), l'adaptateur étant en outre muni d'un premier canal de liaison (306) dans lequel débouche le premier orifice (304) provenant de la première chambre circulaire et d'un deuxième canal de liaison (307) isolé du premier dans lequel débouche le deuxième orifice (305) provenant de la deuxième chambre annulaire, chaque canal (306, 307) comprenant un embout (308, 309) constitué d'une tubulure circulaire, **caractérisé en ce que**
- l'adaptateur est constitué d'une plaque de base (303) de laquelle partent la première (301) et la seconde nervure d'adaptateur (302), le premier orifice (304) et le deuxième orifice (305) étant réalisés dans ladite plaque de base (303),
- les axes des embouts (308, 309) sont parallèles et les extrémités des embouts sont disposées dans un même plan perpendiculaire aux axes des embouts, et
- les axes des embouts sont perpendiculaires à l'axe des nervures concentriques (301, 302),
- lesdits embouts (308, 309) ont des dimensions compatibles avec celles des orifices (201, 202) de la machine de dialyse et sont écartés l'un de l'autre de la même distance prédéfinie que les deux orifices (201, 202) de la machine de dialyse.

2. Adaptateur selon la revendication précédente, **caractérisé en ce que** la première nervure d'adaptateur (301) et la deuxième nervure d'adaptateur (302) sont dimensionnées de telle sorte que lorsque l'adaptateur est monté sur un connecteur concentrique (10), elles viennent s'emboîter sur ou dans des première et deuxième nervures (101, 102) correspondantes du connecteur de sorte que les deux chambres concentriques sont isolées l'une de l'autre et vis-à-vis de l'extérieur.

3. Adaptateur selon l'une des revendications précédentes, **caractérisé en ce que** des moyens de crochetage (310, 311, 312, 313) sont prévus pour en position d'utilisation retenir l'adaptateur sur le connecteur (10).

4. Adaptateur selon la revendication précédente, **caractérisé en ce que** les moyens de crochetage sont constitués par au moins un arceau monté sur des crochets.

5. Adaptateur selon l'une des revendications précédentes, **caractérisé en ce que** les canaux (306, 307) ne sont pas alignés et forment un angle obtus.

6. Adaptateur selon l'une des revendications précédentes, **caractérisé en ce qu'**il est muni d'une poignée.

7. Adaptateur selon l'une des revendications précédentes, **caractérisé en ce qu**'il est muni de moyens de retenue destinés à coopérer avec des moyens complémentaires sur la machine de dialyse pour empêcher l'adaptateur de pivoter sous l'effet du poids lorsqu'il est monté dans la machine.

8. Adaptateur selon la revendication précédente, **caractérisé en ce que** les moyens de retenue sont constitués par une plaque d'appui (330).

9. Adaptateur selon la revendication précédente, **caractérisé en ce que** la plaque d'appui est dimensionnée pour coopérer avec des moyens complémentaires sur la machine de dialyse ayant la forme d'un couvercle (203).

10. Adaptateur selon l'une des revendications précédentes, **caractérisé en ce qu**'il est muni d'une plaque de détection (340) destinée à coopérer avec des moyens de détection complémentaires sur la machine de dialyse.

11. Set constitué
- d'un adaptateur selon l'une des revendications précédentes et
- d'un récipient muni d'un connecteur (10) pour raccorder le récipient à une prise de raccord (2) d'une machine de dialyse, le connecteur (10) étant muni de deux lignes de fluide reliant l'intérieur et l'extérieur du récipient dont les extrémités ouvertes vers l'extérieur sont concentriques.

## Patentansprüche

1. Adapter (3) zum Anschließen eines Behälter-Verbinders (10) an ein Anschlussstück (2) einer Dialysemaschine, wobei der Verbinder (10) mit zwei Fluidleitungen, die den Innenbereich und den Außenbereich des Behälters verbinden, versehen ist, deren nach außen offene Enden konzentrisch sind, wobei das Anschlussstück (2) der Dialysemaschine mit zwei Fluidleitungen versehen ist, deren nach außen offene Enden Öffnungen (201, 202) bilden, die nichtkonzentrisch und voneinander um einen vordefinierten Abstand entfernt sind, wobei der Adapter (3) mit einer ersten (301) und einer zweiten (302) konzentrischen Adapter-Rippe versehen ist, wobei die erste eine nach außen offene Rundkammer bildet, in deren Boden eine erste Öffnung (304) gebildet ist, während die zweite, die um die erste herum angeordnet ist, eine nach außen offene Ringkammer bildet, in deren Boden eine zweite Öffnung (305) gebildet ist, wobei der Adapter außerdem mit einem ersten Verbindungskanal (306), in den die erste Öffnung (304) von der ersten Rundkammer kommend mündet, und mit einem zweiten, von dem ersten getrennten Verbindungskanal (307), in den die zweite Öffnung (305) von der zweiten Ringkammer kommend mündet, versehen ist, wobei jeder Kanal (306, 307) einen Stutzen (308, 309) umfasst, der von einem kreisförmigen Anschlussstutzen gebildet ist, **dadurch gekennzeichnet, dass**
- der Adapter von einer Grundplatte (303) gebildet ist, von der aus sich die erste (301) und die zweite Adapter-Rippe (302) erstrecken, wobei die erste Öffnung (304) und die zweite Öffnung (305) in der Grundplatte (303) gebildet sind,
- die Achsen der Stutzen (308, 309) parallel sind und die Stutzenenden in der gleichen, zu den Achsen der Stutzen senkrechten Ebene angeordnet sind, und
- die Achsen der Stutzen zu der Achse der konzentrischen Rippen (301, 302) senkrecht sind,
- die Stutzen (308, 309) kompatible Abmessungen mit denen der Öffnungen (201, 202) der Dialysemaschine haben und voneinander um den gleichen vordefinierten Abstand entfernt sind wie die zwei Öffnungen (201, 202) der Dialysemaschine.

2. Adapter nach dem vorangehenden Anspruch, **dadurch gekennzeichnet, dass** die erste Adapter-Rippe (301) und die zweite Adapter-Rippe (302) so bemessen sind, dass wenn der Adapter auf einen konzentrischen Verbinder (10) montiert ist, sie sich so auf oder in die korrespondierende erste und zweite Rippe (101, 102) des Verbinders einfügen, dass die zwei konzentrischen Kammern voneinander und nach außen hin isoliert sind.

3. Adapter nach einem der vorangehenden Ansprüche, **dadurch gekennzeichnet, dass** Einhakmittel (310, 311, 312, 313) vorgesehen sind, um in der Gebrauchslage den Adapter auf dem Verbinder (10) festzuhalten.

4. Adapter nach dem vorangehenden Anspruch, **dadurch gekennzeichnet, dass** die Einhakmittel durch mindestens einen Bügel gebildet sind, der auf Haken montiert ist.

5. Adapter nach einem der vorangehenden Ansprüche, **dadurch gekennzeichnet, dass** die Kanäle (306, 307) nicht fluchten und einen stumpfen Winkel bilden.

6. Adapter nach einem der vorangehenden Ansprüche, **dadurch gekennzeichnet, dass** er mit einem Griff versehen ist.

7. Adapter nach einem der vorangehenden Ansprüche, **dadurch gekennzeichnet, dass** er mit Rückhaltemitteln versehen ist, die dazu dienen, mit komplementären Mitteln auf der Dialysemaschine zusammenzuwirken, um zu verhindern, dass der Adapter sich unter der Wirkung des Gewichts dreht, wenn er in der Maschine installiert ist.

8. Adapter nach dem vorangehenden Anspruch, **dadurch gekennzeichnet, dass** die Rückhaltemittel von einer Auflageplatte (330) gebildet sind.

9. Adapter nach dem vorangehenden Anspruch, **dadurch gekennzeichnet, dass** die Auflageplatte zum Zusammenwirken mit komplementären Mittel auf der Dialysemaschine bemessen ist, die die Form eines Deckels (203) aufweisen.

10. Adapter nach einem der vorangehenden Ansprüche, **dadurch gekennzeichnet, dass** er mit einer Erkennungsplatte (340) versehen ist, die dazu dient, mit komplementären Erkennungsmitteln auf der Dialysemaschine zusammenzuwirken.

11. Set, gebildet
- aus einem Adapter nach einem der vorangehenden Ansprüche und
- einem Behälter, der mit einem Verbinder (10) versehen ist, um den Behälter an ein Anschlussstück (2) einer Dialysemaschine anzuschließen, wobei der Verbinder (10) mit zwei Fluidleitungen, die den Innenbereich und den Außenbereich des Behälters verbinden, versehen ist, deren nach außen offene Enden konzentrisch sind.

## Claims

1. Adapter (3) for connecting a receptacle connector (10) to a connection socket (2) of a dialysis machine, the connector (10) being provided with two fluid lines connecting the inside and outside of the receptacle, the ends of which, open towards the outside, are concentric, the connection socket (2) of the dialysis machine being provided with two fluid lines, the ends of which, open towards the outside, form orifices (201, 202) which are non-concentric and distant from each other by a predefined distance, the adapter (3) being provided with concentric first (301) and second (302) adapter ribs, the first forming a circular chamber open towards the outside, in the bottom of which a first orifice (304) is provided, while the second, placed around the first, forms an annular chamber open towards the outside, in the bottom of which a second orifice (305) is provided, the adapter also being provided with a first connection channel (306) in which the first orifice (304) coming from the first circular chamber opens out and a second connection channel (307), isolated from the first, in which the second orifice (305) coming from the second annular chamber opens out, each channel (306, 307) comprising a connecting piece (308, 309) consisting of a circular tube, **characterised in that,**
- the adapter consists in a base plate (303) from which the first (301) and second (302) adapter ribs extend, the first orifice (304) and the second orifice (305) being provided in the said base plate (303),
- the axes of the connecting pieces (308, 309) are parallel and the ends of the connecting pieces are disposed in a same plane perpendicular to the axes of the connecting pieces, and
- the axes of the connecting pieces are perpendicular to the axis of the concentric ribs (301, 302)
- the said connecting pieces (308, 309) have dimensions which are compatible with those of the orifices (201, 202) of the dialysis machine and are distant from each other by the same predefined distance as the two orifices (201, 202) of the dialysis machine.

2. Adapter according to the preceding claim, **characterised in that** the first adapter rib (301) and the second adapter rib (302) are sized so that, when the adapter is mounted on a concentric connector (10), they come to fit on or in the corresponding first and second ribs (101, 102) of the connector so that the two concentric chambers are isolated from each other and vis-à-vis the outside.

3. Adapter according to one of the preceding claims, **characterised in that** hooking means (310, 311, 312, 313) are provided in order to hold the adapter on the connector (10) in the position of use.

4. Adapter according to the preceding claim, **characterised in that** the hooking means are formed by at least one arch mounted on hooks.

5. Adapter according to one of the preceding claims, **characterised in that** the channels (306, 307) are not aligned and form an obtuse angle.

6. Adapter according to one of the preceding claims, **characterised in that it** is provided with a handle.

7. Adapter according to one of the preceding claims, **characterised in that it** is provided with holding means intended to cooperate with complementary means on the dialysis machine for preventing the adapter from pivoting under the effect of weight when it is mounted in the machine.

8. Adapter according to the preceding claim, **characterised in that,** the holding means consist of a support plate (330).

9. Adapter according to the preceding claim, **characterised in that,** the support plate is sized to cooperate with complementary means on the dialysis machine, which complementary means consist of a cover (203).

10. Adapter according to one of the preceding claims, **characterised in that** it is provided with a detection plate (340) intended to cooperate with complementary detection means on the dialysis machine.

11. Set constituted by
- an adapter according to one of the preceding claims, and
- a receptacle provided with a connector (10) for connecting the receptacle to a connecting socket (2) of a dialysis machine, the connector (10) being provided with two fluid lines connecting the inside and the outside of the receptacle, the extremities of which, open to the outside, are concentric.
